# EUROPEAN PATENT APPLICATION

(11) **EP 4 629 133 A1**
(43) Date of publication of application: **08.10.2025**
(21) Application number: 25158001.5
(22) Date of filing: 14.02.2025
(51) Int. Cl.: G06K 7/10, A61B 90/96

(54) **METHOD OF SCANNING MEDICAL INSTRUMENTS**

(30) Priority: 19.03.2024 TW 113110136
(71) Applicant: Liu, Ching-Sen, Taichung City 427 (TW)
(72) Inventor: LIU, Ching-Sen, 427 Taichung City (TW); CHIA, Zong Lin Sprecher, 735788 Singapore (SG)
(74) Representative: Viering, Jentschura & Partner mbB Patent- und Rechtsanwälte

(57) **Abstract**

A method of scanning medical instruments for reading a code (1a) of at least one medical instrument (1) is provided, wherein the code (1a) contains an instrument data corresponding to the at least one medical instrument (1). The method of scanning medical instruments includes capturing a first image of the at least one medical instrument (1) by an image capture unit (21); capturing a second image of the at least one medical instrument (1) by a lateral image capture unit (31), wherein the second image is different from the first image; and providing a process module (2) to receive the first image and the second image, and reading the code (1a) of the at least one medical instrument (1) from the first image and the second image. By replacing manual operations with automated equipment, the efficiency and accuracy of scanning medical instruments (1) are enhanced.

## Description

### BACKGROUND OF THE INVENTION

### Technical Field

The present invention relates generally to a method of using a medical device; and more particularly to a method of scanning medical instruments.

### Description of Related Art

During surgical procedures, a large number and diverse array of medical instruments is typically used for various surgical operations, such as surgical scissors, scalpels, etc. To manage the usage status of the medical instruments, it is common to label a code on surfaces of the medical instruments. After surgery, the medical instruments are then inventoried by comparing the codes of the medical instruments to ensure none of the medical instruments are missing, thus avoiding the situation where the medical instruments are left inside the body of the patient.

However, the conventional way to inventory the medical instruments often relies on visual identification, which is prone to human error. Moreover, manual adjustment of the orientation of the surface of the medical instruments is required during the identification process to bring the codes of the medical instruments to the front for easier recognition. Additionally, due to the limitations of human capabilities, it is challenging to inventory a large number of medical instruments simultaneously. Consequently, the entire inventorying process is time-consuming, which leads to a decrease in overall efficiency and accuracy.

Therefore, there is still room for improvement in conventional techniques for inventorying the medical instruments. Enhancing the efficiency and accuracy of medical instrument scanning is problem needed to be solved in the industry.

### BRIEF SUMMARY OF THE INVENTION

In view of the above, the primary objective of the present invention is to provide a method of scanning medical instruments. By replacing manual operations with automated equipment, the efficiency and accuracy of scanning medical instruments are enhanced.

The present invention provides a method of scanning medical instruments, wherein the method of scanning medical instruments is for reading a code of at least one medical instrument. The code contains an instrument data corresponding to the at least one medical instrument. The method of scanning medical instruments includes capturing a first image of the at least one medical instrument by an image capture unit; capturing a second image of the at least one medical instrument by a lateral image capture unit, wherein the second image is different from the first image; and providing a process module to receive the first image and the second image, and reading the code of the at least one medical instrument from the first image and the second image.

Through the method of scanning medical instruments, the code of the at least one medical instrument could be automatically scanned and compared, thus replacing manual operations to enhance overall efficiency. Additionally, with the design of the image capture unit and the lateral image capture unit, the efficiency and accuracy of scanning medical instruments are enhanced.

### BRIEF DESCRIPTION OF THE SEVERAL VIEWS OF THE DRAWINGS

The present invention would be best understood by referring to the following detailed description of some illustrative embodiments in conjunction with the accompanying drawings, in which
FIG. 1 is a perspective view of the medical instrument scanning device according to an embodiment of the present invention;
FIG. 2 is a schematic view of a part of the components of the medical instrument scanning device according to the embodiment of the present invention;
FIG. 3 is a schematic view of a part of the components of the medical instrument scanning device seen from another perspective according to the embodiment of the present invention;
FIG. 4 is a bottom view of the medical instrument scanning device according to the embodiment of the present invention;
FIG. 5 is a sectional view of along the 5-5 line in FIG. 4, showing a part of the components of the medical instrument scanning device;
FIG. 6 is a schematic view of a part of the components of the medical instrument scanning device according to another embodiment of the present invention;
FIG. 7 is a schematic view of the medical instrument scanning system according to an embodiment of the present invention;
FIG. 8 is a flowchart of the method of scanning medical instruments according to an embodiment of the present invention;
FIG. 9 is a flowchart of the method of scanning medical instruments according to the embodiment of the present invention;
FIG. 10 is a schematic view of the display device of the method of scanning medical instruments, showing that the display device displays the instrument list according to the embodiment of the present invention;
FIG. 11 is a schematic view of the display device of the method of scanning medical instruments, showing that the code data displayed on the display device matches the instrument data, according to the embodiment of the present invention;
FIG. 12 is a schematic view of the display device of the method of scanning medical instruments showing that the display device displays the instrument data of the medical instrument that does not exist in the instrument list according to the embodiment of the present invention;
FIG. 13 is a schematic view of the display device of the method of scanning medical instruments, showing that the display device displays the code data in the comparison result that has not yet been marked with color, according to the embodiment of the present invention.

### DETAILED DESCRIPTION OF THE INVENTION

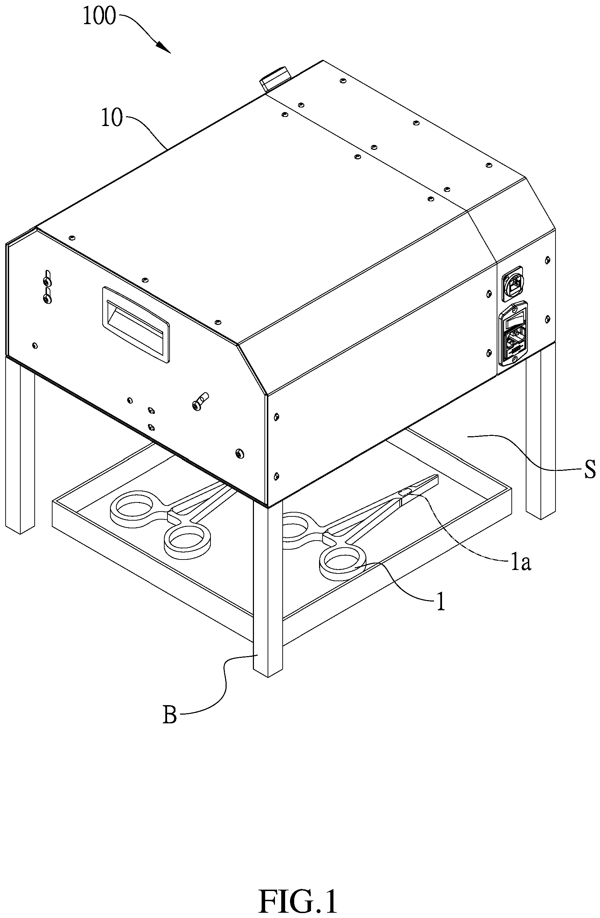

A medical instrument scanning device 100 according to an embodiment of the present invention is illustrated in FIG. 1 to FIG. 5 and is adapted to scan a code 1a of at least one medical instrument 1, wherein the code 1a is located on a surface of the at least one medical instrument 1. The code 1a of the at least one medical instrument 1 could be, for example, a one-dimensional barcode or a two-dimensional barcode engraved or affixed onto the surface of the at least one medical instrument 1. In the current embodiment, the at least one medical instrument 1 is illustrated as a plurality of the medical instruments 1 as an example, and the code 1a is exemplified by a QR code which is formed by recessing into the surface of the medical instrument 1 through engraving.

Referring to FIG. 1 to FIG. 3, the medical instrument scanning device 100 includes a casing 10, a leg part B, a longitudinal read module 20, and a lateral read module 30. The casing 10 has an accommodating space 11 and a lower opening 12, wherein the accommodating space 11 communicates with the lower opening 12. The longitudinal read module 20 and the lateral read module 30 are accommodated in the accommodating space 11. The leg part B is disposed beneath the casing 10 and forms a space S, wherein the space S communicates with both the accommodating space 11 of the casing 10 and the lower opening 12, so that the space S is adapted to accommodate the medical instruments 1 which are scanned.

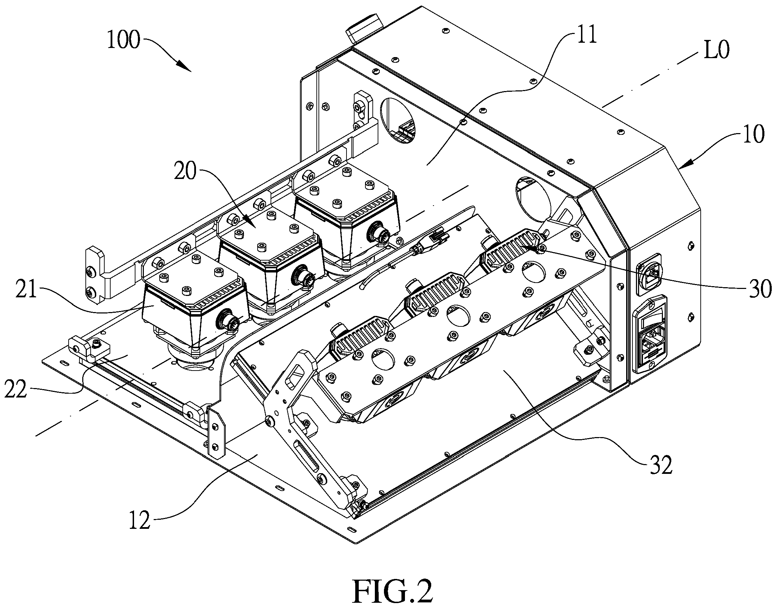

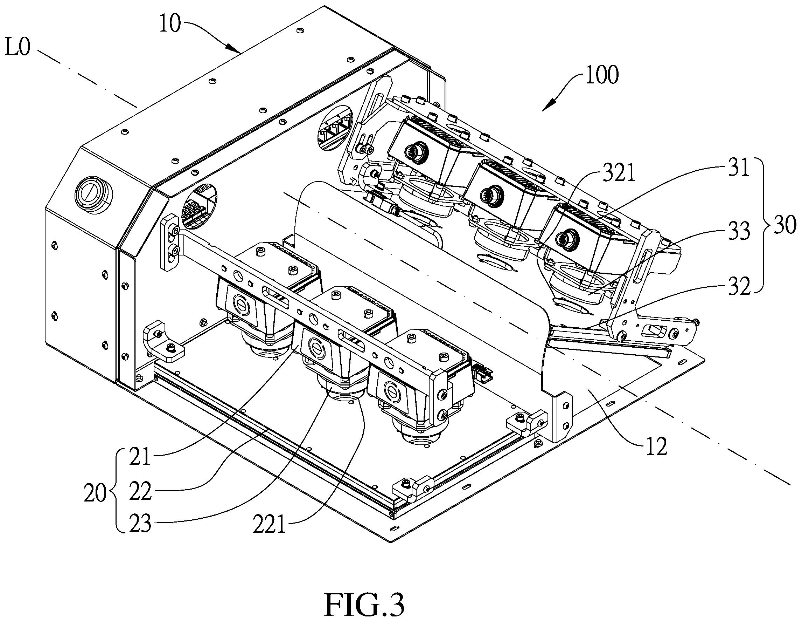

Referring to FIG. 2 and FIG. 3, the medical instrument scanning device 100 defines a lateral axis L0. The longitudinal read module 20 includes a plurality of image capture units 21 and a light source, wherein the image capture units 21 are arranged along the lateral axis L0 and are adapted to capture images of the medical instruments 1.

In the current embodiment, the image capture units 21 are illustrated with three image capture units 21 as an example. By providing multiple image capture units 21, a scanning area is enlarged and thereby a large number of medical instruments could be scanned in a single scan, thus improving the efficiency of medical instrument scanning. In other embodiments, the number of the image capture unit 21 could be one, two, or more, depending on the actual scanning requirements of medical instruments. Besides, the image capture unit 21 could be equipped with a wide-angle lens to expand the field of view as needed. In other words, the number of the image capture unit 21 could be adjusted according to the size of the field of view.

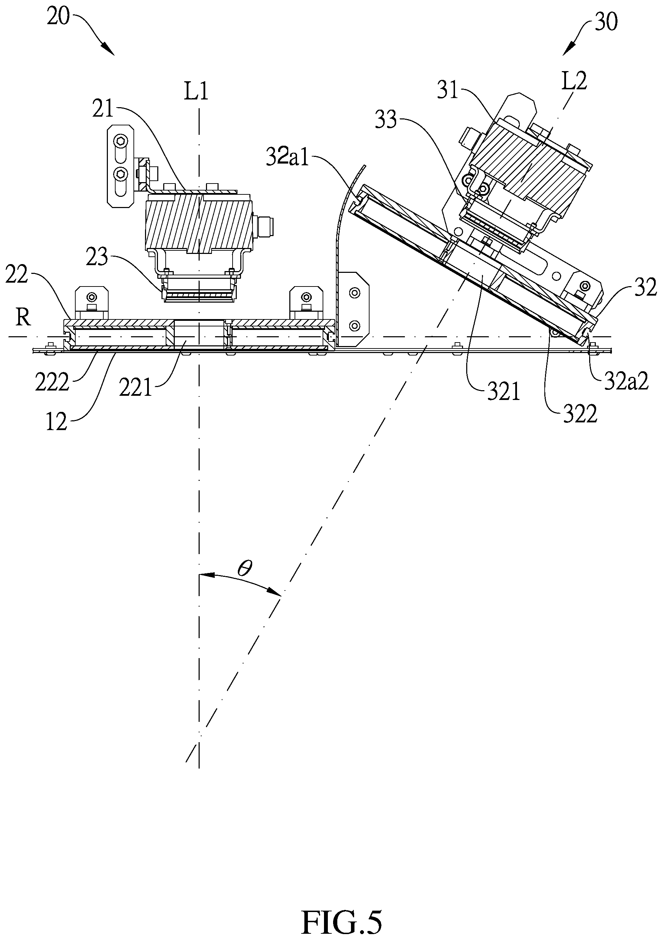

In the current embodiment, the light source is a light board 22, but not limited thereto. In other embodiments, the light source could be a point light source or other light sources. As shown in FIG. 5, the medical instrument scanning device 100 defines a first reference axis L1. The light board 22 is disposed beneath the image capture units 21, and a first distance is provided between the light board 22 and each of the image capture units 21 along the first reference axis L1. Moreover, the light board 22 has a plurality of longitudinal through holes 221, wherein the number and the position of the longitudinal through holes 221 correspond to the number and the position of the image capture units 21. The longitudinal through holes 221 are arranged along the lateral axis L0. In the current embodiment, the number of the longitudinal through holes 221 is three in correspondence to the three image capture units 21.

Referring to FIG. 5, an optical axis of each of the image capture units 21 could correspondingly pass through each of the longitudinal through holes 221 and the lower opening 12 of the casing 10 in a manner parallel to the first reference axis L1, so that the image capture units 21 could capture images of the medical instruments 1 disposed beneath the casing 10. A light axis of the light board 22 could pass through the lower opening 12 of the casing 10 in a manner parallel to the first reference axis L1, and illuminate the surfaces of the medical instruments 1.

Referring to FIG. 5, the light board 22 further includes a first reflection filter 222 disposed on a lower surface of the light board 22. The first reflection filter 222 allows the light of the light board 22 to pass through the lower opening 12 of the casing 10 in a manner parallel to the first reference axis L1, and illuminate the surfaces of the medical instruments 1. This prevents the reflected light generated by the medical instruments 1 from affecting the images captured by the image capture unit 21.

Referring to FIG. 2 and FIG. 3, in the current embodiment, the longitudinal read module 20 includes a plurality of second reflection filters 23. The number of the second reflection filters 23 is three in correspondence to the number of the image capture units 21. Each of the second reflection filters 23 is disposed between each of the image capture units 21 and the light board 22, so that the second reflection filters 23 filter out the reflected light from the metal surfaces of the medical instruments 1, which is generated by the light emitted from the light board 22. Thereby, the design of the second reflection filters 23 enhances the resolution of the images captured by the image capture units 21 and improves the accuracy of code reading.

Referring to FIG. 2 and FIG. 3, the lateral read module 30 includes a plurality of lateral image capture units 31 and a lateral light source. The lateral image capture units 31 are arranged along the lateral axis L0 and are adapted to capture images of the medical instruments 1. In the current embodiment, the number of the lateral image capture units 31 is three, but not limited thereto. In other embodiments, the number of the lateral image capture unit 31 could be one, two, or more. By providing multiple lateral image capture units 31, the scanning area could be enlarged and thereby a large number of medical instruments could be scanned in a single scan, thus improving the efficiency of medical instrument scanning. Besides, the lateral image capture units 31 could be equipped with the wide-angle lens to expand the field of view as needed. In other words, the number of the lateral image capture unit 31 could be adjusted according to the size of the field of view.

Moreover, in the current embodiment, the number and the position of the image capture units 21 correspond to the number and the position of the lateral image capture units 31. In other embodiments, the number and the position of the image capture units 21 and the number and the position of the lateral image capture units 31 could be independently adjusted to meet different requirements.

In the current embodiment, the lateral light source is a lateral light board 32, but not limited thereto. In other embodiments, the lateral light source could be a point light source, etc. As shown in FIG. 5, the medical instrument scanning device 100 defines a second reference axis L2. The lateral light board 32 is disposed beneath the lateral image capture units 31, and a second distance is provided between the lateral light board 32 and each of the lateral image capture units 31 along the second reference axis L2. Moreover, the lateral light board 32 has a plurality of lateral through holes 321 corresponding to the image capture units 31. The lateral through holes 321 are arranged along the lateral axis L0. In the current embodiment, the number of the lateral through holes 321 is three in correspondence to the number of the image capture units 31.

Referring to FIG. 5, an optical axis of each of the lateral image capture units 31 could correspondingly pass through each of the lateral through holes 321 and the lower opening 12 of the casing 10 in a manner parallel to the first reference axis L1, so that the lateral image capture units 31 could capture images of the medical instruments 1 disposed beneath the casing 10. A light axis of the lateral light board 32 could pass through the lower opening 12 of the casing 10 in a manner parallel to the second reference axis L2, and illuminate the surfaces of the medical instruments 1.

Moreover, the medical instrument scanning device 100 defines a lateral reference plane R which passes through the light board 22 and is parallel to the light board 22. The lateral light board 32 has a first side 32a1 and a second side 32a2 that are opposite to each other. The first side 32a1 is closer to the light board 22 compared to the second side 32a2, and the first side 32a1 of the lateral light board 32 is farther from the lateral reference plane R compared to the second side 32a2. In other words, the light axis of the lateral light board 32 is not parallel to the light axis of the light board 22.

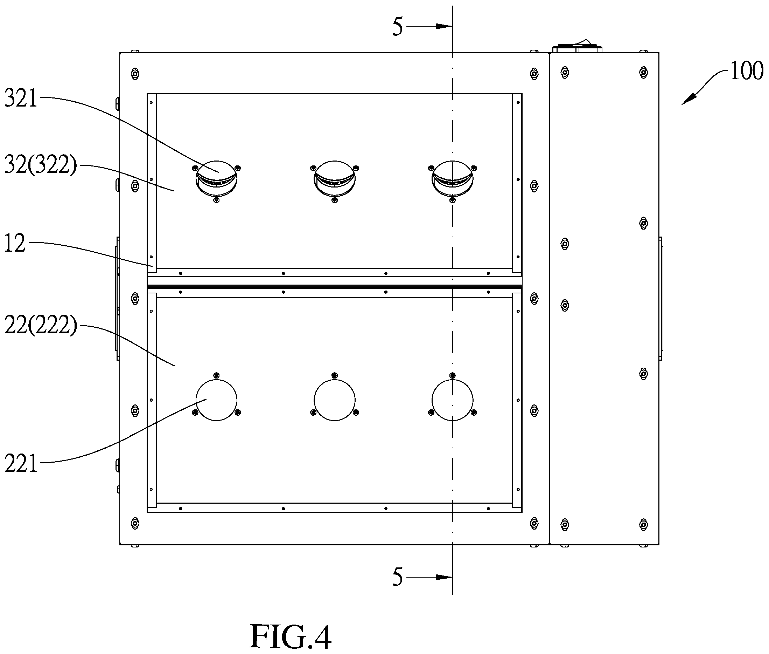

As shown in FIG. 4 and FIG. 5, the lateral read module 30 is disposed next to the longitudinal read module 20, and an angle θ is provided between the first reference axis L1 and the second reference axis L2. In other words, the angle θ is between the optical axis of each of the image capture units 21 and the optical axis of each of the lateral image capture units 31. The angle θ is greater than 0 degree and is less than or equal to 60 degrees. Preferably, the angle θ could be greater than or equal to 15 degrees and be less than or equal to 45 degrees. In the current embodiment, the angle θ is illustrated as 30 degrees as an example. Thereby, the lateral read module 30 and the longitudinal read module 20 could capture images of the medical instruments 1 from different directions. In this way, when the medical instruments 1 are not placed flat or are tilted at different angles, the medical instrument scanning device 100 could still accurately read the code 1a on each of the medical instruments 1 by the images captured by both the longitudinal read module 20 and the lateral read module 30, thereby enhancing the scanning capability of the medical instrument scanning device 100.

Referring to FIG. 4, the lateral light board 32 further includes a first reflection filter 322 disposed on a lower surface of the lateral light board 32. The first reflection filter 322 allows the light of the lateral light board 32 to pass through the lower opening 12 of the casing 10 in a manner parallel to the second reference axis L2, and illuminate the surfaces of the medical instruments 1. This prevents the reflected light generated by the medical instruments 1 from affecting the images captured by the lateral image capture unit 31.

Referring to FIG.2 and FIG. 3, in the current embodiment, the lateral read module 30 includes a plurality of second reflection filters 33. The number of the second reflection filters 33 correspond to the number of the lateral image capture units 31. In the current embodiment, the number of the second reflection filters 33 is three in correspondence to the number of the lateral image capture units 31. The second reflection filters 33 are disposed between the lateral image capture units 31 and the lateral light board 32, so that the second reflection filters 33 filter out the reflected light from the metal surfaces of the medical instruments 1. Thereby, the design of the second reflection filters 33 enhances the resolution of the images captured by the lateral image capture units 31 and improves the accuracy of code reading.

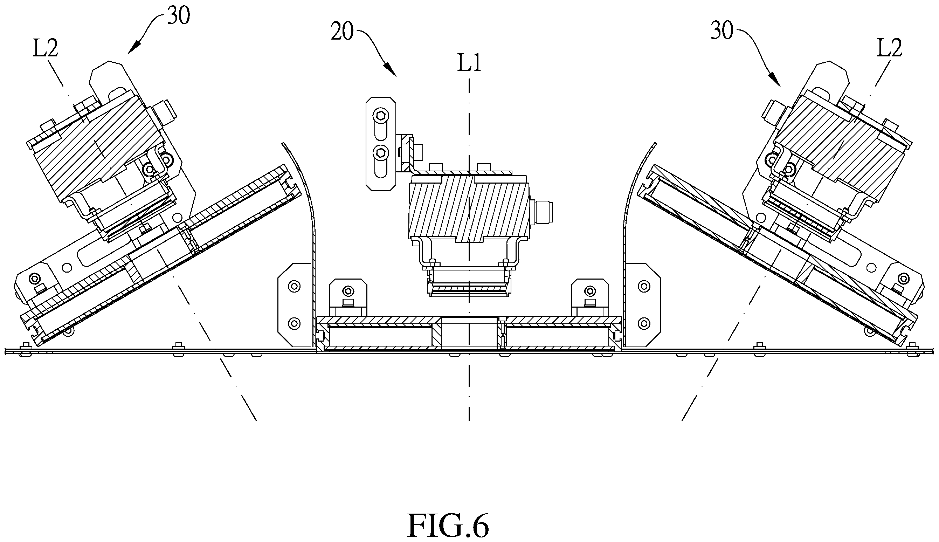

In the current embodiment, the number of the lateral read module 30 is illustrated as one as an example. In other embodiments, the number of the lateral read module 30 could also be plural. For example, the plurality of lateral read modules 30 could be arranged around the longitudinal read module 20, thereby capturing images of the medical instruments 1 form more different directions to have a wider field of view, thus enhancing the scanning capability of the medical instrument scanning device 100. As shown in FIG. 6, in another embodiment, the number of the lateral read module 30 is two. The two lateral read modules 30 are disposed next to the longitudinal read module 20 and are respectively disposed on two opposite sides of the longitudinal read module 20. In this way, when the medical instruments 1 are not placed flat or are tilted at different angles, the medical instrument scanning device 100 could still accurately read the code 1a on each of the medical instruments 1 by the images captured by both the longitudinal read module 20 and the lateral read modules 30.

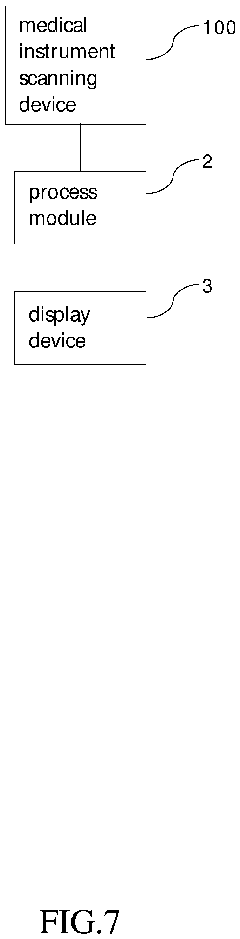

A medical instrument scanning system according to an embodiment of the present invention is illustrated in FIG. 7 and includes the medical instrument scanning device 100 of the aforementioned embodiment, a process module 2, and a display device 3. The process module 2 is electrically connected to both the medical instrument scanning device 100 and the display device 3.

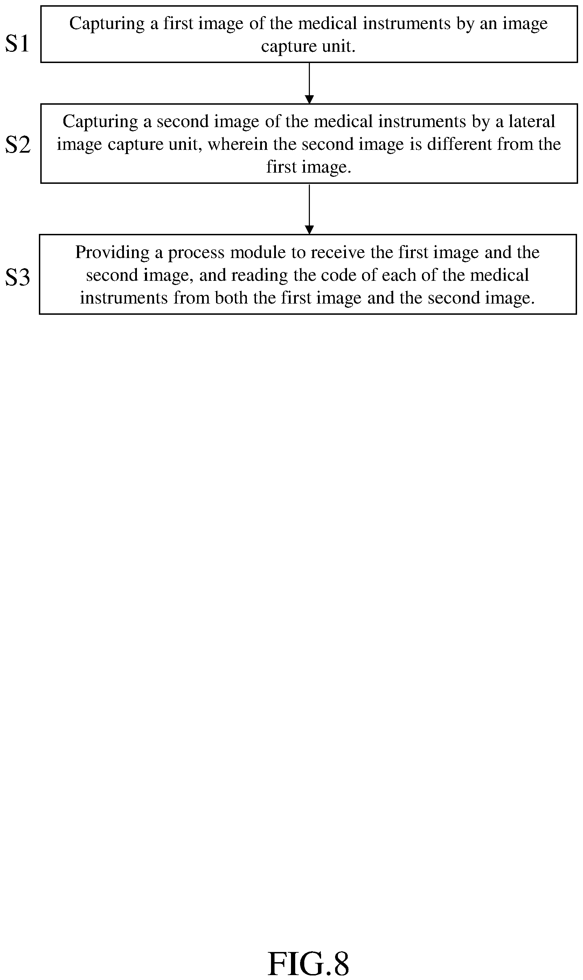

A method of scanning medical instruments according to an embodiment of the present invention is illustrated in FIG. 8. In the current embodiment, the method of scanning medical instruments is executed by the aforementioned medical instrument scanning system as an example. In practice, the method of scanning medical instruments could also be executed by other devices, which means that the method of scanning medical instruments is not limited to being executed by the aforementioned medical instrument scanning system. The code 1a of the each of the medical instruments 1 contains an instrument data corresponding to each of the medical instruments 1. In the current embodiment, the instrument data could include a name, a serial number, a placement location, and other information of each of the medical instruments 1. The method of scanning medical instruments includes the following steps.

Step S1: capturing a first image of the medical instruments 1 by an image capture unit. In the current embodiment, the image capture unit is the image capture unit 21 of the aforementioned embodiment. In order to illustrate easily, the number of the image capture unit 21 in the current embodiment is illustrated as one as an example. In other embodiments, the number of the image capture unit 21 could also be one, two, or more. In the current embodiment, the number of the first image corresponds to the number of the image capture unit 21. In practice, it is necessary to first place the medical instruments 1 in the space S, which is located at the bottom of medical instrument scanning device 100, to prepare for scanning.

Step S1 further includes providing the light source. Both the optical axis of the image capture unit 21 and the light axis of the light source are parallel to the first reference axis L1. The light source emits light along the first reference axis L1 and illuminates the surfaces of the medical instruments 1.

Step S2: capturing a second image of the medical instruments 1 by a lateral image capture unit, wherein the second image is different from the first image. In the current embodiment, the lateral image capture unit is the lateral image capture unit 31 of the aforementioned embodiment. In order to illustrate easily, the number of the lateral image capture unit 31 in the current embodiment is illustrated as one as an example. In practice, the number of the lateral image capture unit 31 could also be one, two, or more. In the current embodiment, the number of the second image corresponds to the number of the lateral image capture unit 31. Alternatively, the number of the second image could be determined by configuring a relevant parameter of the lateral image capture unit 31, wherein the relevant parameter is the number of the second image captured by the lateral image capture unit 31 over a period of time.

Step S2 further includes providing the lateral light source. Both the optical axis of the lateral image capture unit 31 and the light axis of the lateral light source are parallel to the second reference axis L2. The lateral light source emits light along the second reference axis L2 and illuminates the surfaces of the medical instruments 1. The execution order of step S1 and step S2 is not limited to executing step S1 before step S2, as shown in the current embodiment. Step S2 could be executed before step S1 or step S1 and step S2 could be executed simultaneously.

With the design of the angle θ provided between the first reference axis L1 and the second reference axis L2, which means that the optical axis of the lateral image capture unit 31 is not parallel to the optical axis of the image capture unit 21, the lateral image capture unit 31 and the image capture unit 21 could capture images of the medical instruments 1 to be scanned from different directions. Thereby, the second image captured by the lateral image capture unit 31 is different from the first image captured by the image capture unit 21. The angle θ is greater than 0 degree and is less than or equal to 60 degrees. Preferably, the angle θ could be greater than or equal to 15 degrees and be less than or equal to 45 degrees. In the current embodiment, the angle θ is illustrated as 30 degrees as an example.

Step S3: providing a process module 2 to receive the first image and the second image, and reading the code 1a of each of the medical instruments 1 from both the first image and the second image. The process module 2 is electrically connected to both the image capture unit 21 and the lateral image capture unit 31. The process module 2 recognizes the image of the code 1a in both the first image and the second image and reads the code 1a to obtain the instrument data corresponding to the medical instrument 1.

The method of scanning medical instruments includes providing an instrument list 3a, wherein the instrument list 3a includes at least one code data D. The process module 2 reads the instrument data from the code 1a and compares the instrument data with the at least one code data D to generate a comparison result. In the current embodiment, the instrument list 3a could be obtained by connecting an external storage device such as a USB drive, a hard disk, etc. In other embodiments, the number of the instrument list 3a and the number of the at least one code data D could be established according to the requirements of different surgeries. The at least one code data D could include information such as a name, a serial number, a placement location, and a quantity of the medical instrument 1 required for the surgery. For example, multiple instrument lists 3a could be established to correspond to the requirements of different surgeries for user selection, and each of the instrument lists 3a could include multiple code data D that are different from one another. Each of the code data D includes the information such as a name, a serial number, a placement location, and a quantity of the corresponding medical instrument 1.

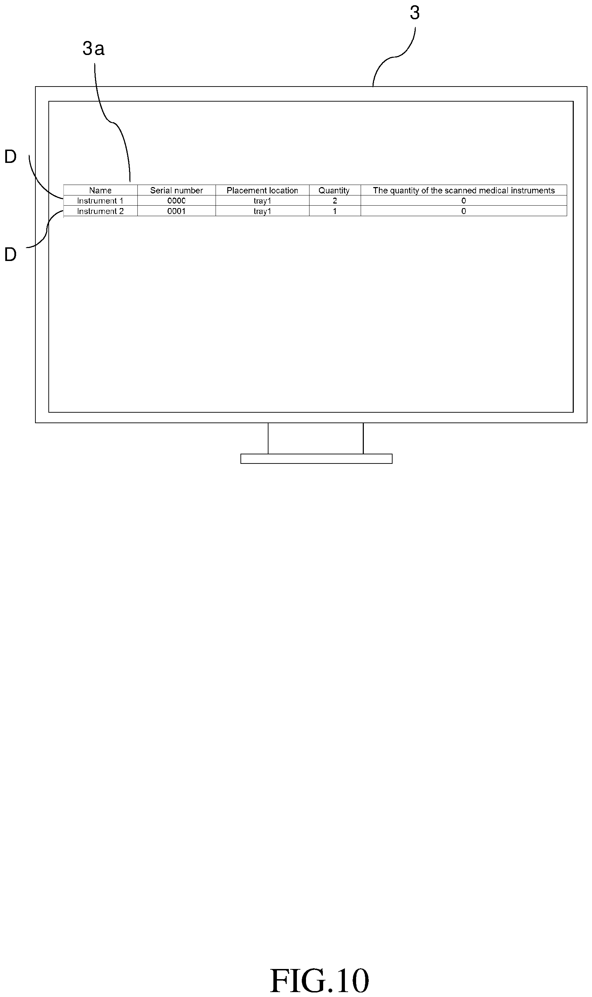

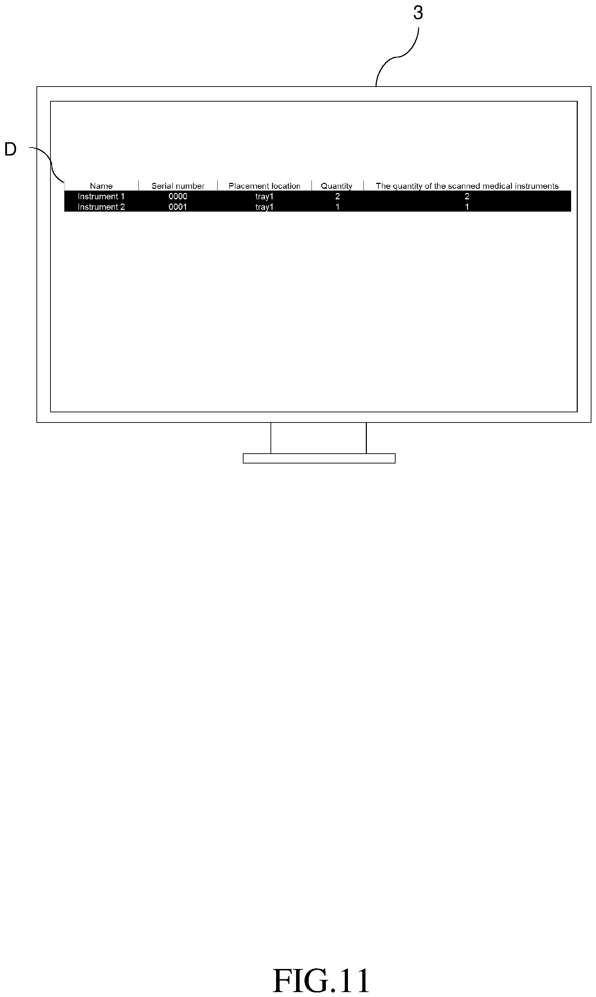

The method of scanning medical instruments includes providing a display device 3 to display the comparison result. The comparison result includes the at least one code data D. When the instrument data matches the at least one code data D, a mark is displayed at a location of the matched code data D. The mark could be made by using different colors or by different ways. For example, as shown in FIG. 10, multiple code data D of an instrument list 3a are displayed on the display device 3 for user to view, and the code data D have not yet been marked with color. When the process module 2 reads the code 1a of each of the medical instruments 1 from both the first image and the second image to obtain the instrument data corresponding to each of the medical instruments 1, the process module 2 compares the code data D with the instrument data of the medical instruments 1. When one of the code data D matches the instrument data, a color mark is displayed at a location of the matched code data D as shown in FIG. 11. Thereby, a user could clearly determine whether the medical instruments 1 in the instrument list 3a are indeed located in a carrier.

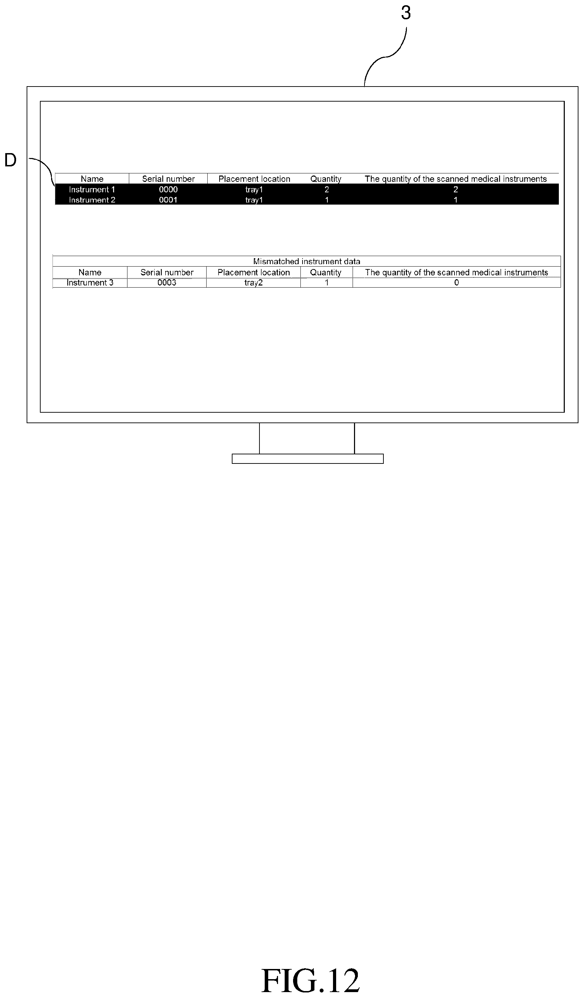

Moreover, the method of scanning medical instruments includes displaying, by the display device 3, the instrument data when the instrument data does not match the at least one code data D. For example, when a medical instrument 1 that does not exist in the instrument list 3a appears in the carrier and correspondingly the instrument data does not match the code data D, the display device 3 displays the instrument data of the medical instrument 1 that does not exist in the instrument list 3a as shown in FIG. 12. In this way, the user could clearly know the instrument data of the medical instrument 1 that should not exist in the carrier below, so that the user could remove the medical instrument 1 that should not exist.

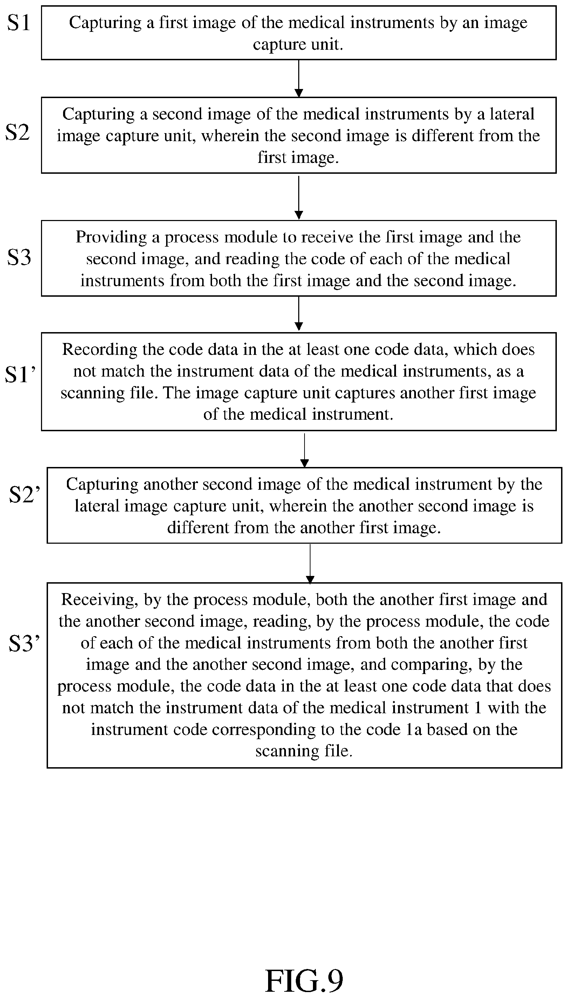

Referring to FIG. 9, the method of scanning medical instruments further includes the following steps:
Step S1': recording the code data D in the at least one code data D, which does not match the instrument data of the medical instruments 1, as a scanning file. The image capture unit 21 captures another first image of the medical instrument 1.
Step S2': capturing another second image of the medical instrument 1 by the lateral image capture unit 31, wherein the another second image is different from the another first image.
Step S3': receiving, by the process module 2, both the another first image and the another second image, reading, by the process module 2, the code 1a of each of the medical instruments 1 from both the another first image and the another second image, and comparing, by the process module 2, the code data D in the at least one code data D that does not match the instrument data of the medical instrument 1 with the instrument code corresponding to the code 1a based on the scanning file.

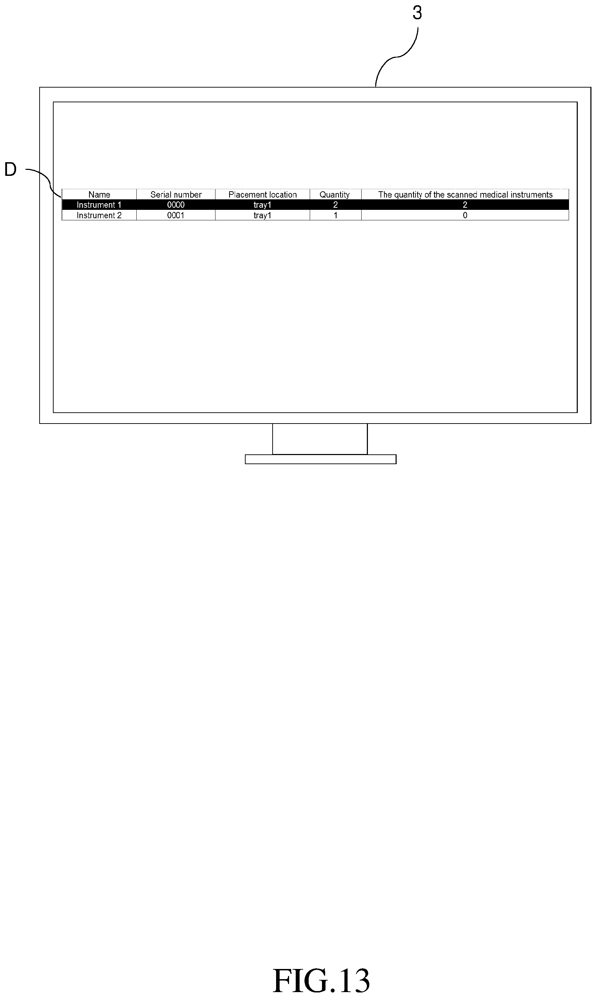

For example, as shown in FIG. 13, when the comparison result displayed by the display device 3 still contains the code data D that has not yet been marked with color, the process module 2 could record the code data D in the at least one code data D that does not match the instrument data of the medical instruments 1 as the scanning file. Then, the user adjusts the placement or the orientation of the medical instruments 1, and places the medical instruments 1 into the space S of the medical instrument scanning device 100 for scanning again. Next, the process module 2 reads the code 1a from both the another first image and the another second image, and compares the code data D that has not been marked with color with the instrument data of the corresponding code 1a based on the scanning file. When the code data D matches the instrument data, the color mark is displayed at the location of the matched code data D as described above. The user could repeat the aforementioned steps until all code data D in the instrument list 3a have been marked with color.

In summary, with the design of the angle θ provided between the first reference axis L1 and the second reference axis L2, the medical instrument scanning device 100 could capture images of contours the medical instruments 1 to be scanned from different directions. In this way, when the medical instruments 1 are not placed flat or are tilted at different angles, the code 1a on each of the medical instruments 1 could still be accurately read from the images captured by both the longitudinal read module 20 and the lateral read module 30. In this way, the scanning capability of the medical instrument scanning device 100 could be enhanced and the efficiency and the accuracy of inventorying the medical instruments could be improved. With the method of scanning medical instruments, the code 1a of the at least one medical instrument 1 could automatically be scanned and compared, thus replacing manual operations to enhance overall efficiency. Moreover, with the disposal of the longitudinal read module 20 and the lateral read module 30, the efficiency and the accuracy of medical instrument scanning could be further enhanced.

## Claims

1. A method of scanning medical instruments for reading a code (1a) of at least one medical instrument (1), wherein the code (1a) contains an instrument data corresponding to the at least one medical instrument (1); the method of scanning medical instruments (1) comprising:
capturing a first image of the at least one medical instrument (1) by an image capture unit (21);
capturing a second image of the at least one medical instrument (1) by a lateral image capture unit (31), wherein the second image is different from the first image; and
providing a process module (2) to receive the first image and the second image, and reading the code (1a) of the at least one medical instrument (1) from the first image and the second image.

2. The method of scanning medical instruments as claimed in claim 1, further comprising providing an instrument list (3a), wherein the instrument list (3a) comprises at least one code data (D); the process module (2) reads the instrument data from the code (1a) and compares the instrument data with the at least one code data (D) to generate a comparison result.

3. The method of scanning medical instruments as claimed in claim 2, further comprising providing a display device (3) to display the comparison result, wherein the comparison result comprises the at least one code data (D); when the instrument data matches the at least one code data (D), a mark is displayed at a location of the at least one code data (D) that is matched.

4. The method of scanning medical instruments as claimed in claim 3, further comprising displaying, by the display device (3), the instrument data when the instrument data does not match the at least one code data (D).

5. The method of scanning medical instruments as claimed in claim 2, further comprising:
recording one of the at least one code data (D), which does not match the instrument data of the at least one medical instrument (1), as a scanning file; capturing another first image of the at least one medical instrument (1) by the image capture unit (21);
capturing another second image of the at least one medical instrument (1) by the lateral image capture unit (31), wherein the another second image is different from the another first image; and
receiving, by the process module (2), both the another first image and the another second image, reading, by the process module (2), the code (1a) of the at least one medical instrument (1) from both the another first image and the another second image, and comparing, by the process module (2), the code data (D) in the at least one code data (D) that does not match the instrument data of the at least one medical instrument (1) with the instrument code corresponding to the code (1a) based on the scanning file.

6. The method of scanning medical instruments as claimed in claim 2, further comprising storing a plurality of instrument lists (3a), wherein each of the instrument lists (3a) comprises a plurality of code data (D) that are different from one another.

7. The method of scanning medical instruments as claimed in claim 1, further comprising providing a light source and a lateral light source, wherein a first reference axis (L1) and a second reference axis (L2) are defined; an optical axis of the image capture unit (21) and a light axis of the light source are parallel to the first reference axis (L1); an optical axis of the lateral image capture unit (31) and a light axis of the lateral light source are parallel to the second reference axis (L2); the light source and the lateral light source respectively emit light along the first reference axis (L1) and the second reference axis (L2) and illuminate a surface of the at least one medical instrument (1); an angle (θ) is provided between the first reference axis (L1) and the second reference axis (L2).

8. The method of scanning medical instruments as claimed in claim 7, wherein the angle (θ) is greater than 0 degree and is less than or equal to 60 degrees.

9. The method of scanning medical instruments as claimed in claim 8, wherein the angle (θ) is greater than or equal to 15 degrees and is less than or equal to 45 degrees.

10. The method of scanning medical instruments as claimed in claim 7, wherein a first distance is provided between the light source and the image capture unit (21), and a second distance is provided between the lateral light source and the lateral image capture unit (31).
